# EUROPEAN PATENT SPECIFICATION

(11) **EP 1 661 578 B1**
(45) Date of publication and mention of the grant of the patent: **14.03.2018**
(21) Application number: 04772317.6
(22) Date of filing: 27.08.2004
(51) Int. Cl.: A61K 38/18, A61K 9/70, A61P 17/02, A61P 9/00, A61P 43/00

(54) **SKIN ULCER PREVENTIVE CURATIVE AGENT CONTAINING HUMAN RECOMBINANT HGF**
MITTEL ZUR PRÄVENTION UND HEILUNG VON HAUTGESCHWÜREN MIT HUMANEM REKOMBINANTEM HGF
AGENT CURATIF ET PREVENTIF DE L'ULCERE DE LA PEAU CONTENANT UN HGF DE RECOMBINAISON HUMAIN

(30) Priority: 03.09.2003 JP 2003311936
(43) Date of publication of application: 31.05.2006
(73) Proprietor: Nakamura, Toshikazu, Kyoto 606-8333 (JP); Kringle Pharma Inc., Osaka 567-0085 (JP)
(72) Inventor: NAKAMURA, Toshikazu, Kyoto-shi, Kyoto 606-8333 (JP); YOSHIDA, Saho, Osaka 562-0043 (JP); MATSUMOTO, Kunio, Ishikawa 920-1192 (JP); ITAMI, Satoshi, Kawanishi-shi, Hyogo 666-0145 (JP); YOSHIKAWA, Kunihiko, Toyono-gun, Osaka 563-0103 (JP)
(74) Representative: Witte, Weller & Partner Patentanwälte mbB
(86) International application number: PCT/JP2004/012361
(87) International publication number: WO 2005/023287

(56) References cited:
- EP-A1- 0 461 560
- EP-A1- 0 462 277
- EP-A1- 0 550 769
- EP-A2- 0 492 614
- WO-A-00/78259
- WO-A1-94/14845
- JP-A- 7 179 356
- US-A- 5 821 223
- TOYODA, M. ET AL: 'Overexpression of hepacyte growth factor/scatter factor promotes vascularizatio n and granulation tissue formation in vivo' FEBS LETTER vol. 509, 2001, pages 95 - 100, XP004329151
- SEKI, T. ET AL: 'Isolation and expression of cdna for different forms of hepatocyte growth factorfrom human leukocyte' BIOCHEMICAL AND BIOPHYSICAL RESEARCH COMMUNICATIONS vol. 172, no. 1, 1990, pages 321 - 327, XP002084490
- FRANKLIN H. EPSTEIN: "Cutaneous wound healing", THE NEW ENGLAND JOURNAL OF MEDICINE, 1999, pages 738-746,
- S. YOSHIDA ET AL: "Recombinant Hepatocyte Growth Factor Accelerates Cutaneous Wound Healing in a Diabetic Mouse Model", GROWTH FACTORS, vol. 22, no. 2, June 2004 (2004-06), pages 111-119,
- N. SHIMA ET AL: "Hepatocyte Growth Factor and its Variant with a Deletion of Five Amino Acids are Distinguishable in their Biological Activity and Tertiary Structure", BIOCHEMICAL AND BIOPHYSICAL RESEARCH COMMUNICATIONS, vol. 200, no. 2, 1994, pages 808-815,
- Y. SHIRIKATA ET AL: SAIBOUZOUSYOKUINSI TO SAISEIIRYOU, 2006, pages 126-130,

## Description

### Technical Field

The present invention relates to a a neovascularization promoting agent and a granulation formation-promoting agent comprising a human recombinant HGF or a gene encoding a human recombinant HGF, wherein five amino acid residues are deleted in the first Kringle domain.

### Background Art

Various growth factors are involved in wound healing of skin by controlling proliferation of cells, migration of epidermal cells, apoptosis (cell death) or extracellular matrix production in the skin. It has been also reported that cutaneous wound healing in a normal animal is promoted by administration of a certain growth factor, or a growth factor compensates for reduction in the wound healing ability of the skin in a diabetic patient who has the reduced ability in wound healing of skin, or a model animal for steroid administration or malnutrition (non-patent literature 1, non-patent literature 2). For example, in a diabetic mouse, it has been reported that cutaneous wound healing is delayed as compared with a normal mouse, but local administration of basic fibroblast growth factor (bFGF), platelet-derived growth factor, insulin-like growth factor-I or transforming growth factor- suppresses delay of the cutaneous wound healing (non-patent literatures 4 to 6). Furthermore, recombinant bFGF is utilized as a therapeutic for a skin ulcer.

On the other hand, HGF (hepatocyte growth factor) which was found as a growth promoting factor for mature hepatocytes has the physiological function responsible for regeneration and protection of the tissue on various damages (non-patent literatures 7 to 10). Also in the skin, it has been elucidated that HGF promotes proliferation or migration of epidermal keratinocyte or melanocyte, and it has been suggested that HGF is involved in the physiological function or wound healing of the skin (non-patent literatures 11 to 13). The present inventors further continued to study a role of HGF in the physiological function and wound healing of the skin, developed a wound therapeutic or a skin ulcer therapeutic agent containing a recombinant HGF as an effective ingredient, and obtained a patent (patent literature 1). The patent literature 1 (corresponding to EP 0492614) discloses that HGF accelerates growth of epitheliocytes. Also in the study thereafter, it has been supported that HGF is involved in cutaneous wound healing in an experimental animal. For example, expression of HGF and a c-Met/HGF receptor is increased depending on skin wounds (non-patent literature 14, non-patent literature 15). In a transgenic mouse overexpressing HGF, acceleration of neovascularization and formation of a granulation tissue after skin wound is seen (non-patent literature 16). On the other hand, in a cutaneous wound healing model, when the action of endogenous HGF is blocked by administration of an anti-HGF antibody, epidermis regeneration and neovascularization are suppressed, and healing is delayed (non-patent literature 15).

However, the aforementioned literatures neither describe nor suggest human recombinant HGF wherein five amino acid residues are deleted in the first Kringle domain.

A human recombinant HGF wherein five amino acid residues are deleted in the Kringle domain is the known protein (non-patent literature 17), but the non-patent literature 17 neither describe nor suggest the action of promoting granulation formation and neovascularization.

In addition, it is described that an endogenous HGF is involved in granulation formation and neovascularization (non-patent literatures 15, 16), but whether or not such exogenous HGF causes the same phenomenon when HGF is administered is not studied at all, and a human recombinant HGF wherein five amino acid residues are deleted in the first Kringle domain is neither described nor suggested.

### (Patent literature 1)

Japanese Patent No. 3200609

### (Non-patent literature 1)

Suh, D.Y., Hunt, T.K. and Spencer, E.M., "Insulin-like growth factor-I reverses the impairment of wound healing induced by corticosteroids in rats.", Endocrinology, 131, p.2399-2403 (1992).

### (Non-patent literature 2)

Albertson, S. , Hummel, R.P. , 3rd, Breeden, M. and Greenhalgh, D.G., "PDGF and FGF reverse the healing impairment in protein-malnourished diabetic mice." , Surgery, 114, p.368-372; discussion p.372-363 (1993).

### (Non-patent literature 3)

Greenhalgh, D.G., Sprugel, K.H., Murray, M.J. and Ross, R., "PDGF and FGF stimulate wound healing in the genetically diabetic mouse.", The American Journal of Pathology, 136, p.1235-1246 (1990).

### (Non-patent literature 4)

Tsuboi, R. and Rifkin, D.B., "Recombinant basic fibroblast growth factor stimulates wound healing in healing-impaired db/db mice. " , Journal of Experimental Medicine, 172, p.245-251 (1990).

### (Non-patent literature 5)

Brown, R.L., Breeden, M.P. and Greenhalgh, D.G., "PDGF and TGF-alpha act synergistically to improve wound healing in the genetically diabetic mouse.", Journal of Surgical Research, 56, p.562-570 (1994).

### (Non-patent literature 6)

Tsuboi, R., Shi, C.M., Sato, C., Cox, G.N. and Ogawa, H., "Co-administration of insulin-like growth factor (IGF)-I and IGF-binding protein-1 stimulates wound healing in animal models.", Journal of Investigative Dermatology, 104, p.199-203 (1995).

### (Non-patent literature 7)

Nakamura, T., Nishizawa, T., Hagiya, M., Seki, T., Shimonishi, M., Sugimura, A., Tashiro, K. and Shimizu, S., "Molecular cloning and expression of human hepatocyte growth factor.", Nature, 342, p.440-443 (1989).

### (Non-patent literature 8)

Boros, P. and Miller, C.M., "Hepatocyte growth factor: a multifunctional cytokine.", Lancet, 345, p.293-295 (1995).

### (Non-patent literature 9)

Zarnegar, R. and Michalopoulos, G.K., "The many faces of hepatocyte growth factor: from hepatopoiesis to hematopoiesis.", Journal of Cell Biology, 129, p.1177-1180 (1995).

### (Non-patent literature 10)

Matsumoto, K. and Nakamura, T., "Hepatocyte growth factor: renotropic role and potential therapeutics for renal diseases . " , Kidney International, 59, p.2023-2038 (2001).

### (Non-patent literature 11)

Matsumoto, K., Hashimoto, K., Yoshikawa, K. and Nakamura, T., "Marked stimulation of growth and motility of human keratinocytes by hepatocyte growth factor.", Experimental Cell Research, 196, p.114-120 (1991).

### (Non-patent literature 12)

Matsumoto, K., Tajima, H. and Nakamura, T., "Hepatocyte growth factor is a potent stimulator of human melanocyte DNA synthesis and growth.", Biochemical and Biophysical Research Communications, 176, p.45-51 (1991).

### (Non-patent literature 13)

Halaban, R., Rubin, J.S., Funasaka, Y. , Cobb, M. , Boulton, T., Faletto, D., Rosen, E., Chan, A., Yoko, K., White, W. and et al., "Met and hepatocyte growth factor/scatter factor signal transduction in normal melanocytes and melanoma cells.", Oncogene, 7, p.2195-2206 (1992).

### (Non-patent literature 14)

Cowin.A.J.,Kallincos,N.,Hatzirodos,N.,Robertson,J.G., Pickering, K.J. , Couper, J. andBelford, D.A. , "Hepatocyte growth factor and macrophage-stimulating protein are upregulated during excisional wound repair in rats.", Cell and Tissue Research, 306, p.239-250 (2001).

### (Non-patent literature 15)

Yoshida, S., Yamaguchi, Y., Itami, S., Yoshikawa, K., Tabata, Y. , Matsumoto, K. andNakamura, T. , "Neutralization of hepatocyte growth factor leads to retarded cutaneous wound healing associated with decreased neovascularization and granulation tissue formation.", Journal of Investigative Dermatology, 120, p.335-343 (2003).

### (Non-patent literature 16)

Toyoda, M., Takayama, H., Horiguchi, N., Otsuka, T., Fukusato, T., Merlino, G., Takagi, H. and Mori, M., "Overexpression of hepatocyte growth factor/scatter factor spurs vascularization and granulation tissue formation in vivo. " , FEBS Letters, 509, p.95-100 (2001).

### (Non-patent literature 17)

Seki, T. , Ihara, I. , Sugimura, A. , Shimonishi, M. , Nishizawa, T., Asami, O., Hagiya, M., Nakamura, T. and Shimizu, S., "Isolation and expression of cDNA for different forms of hepatocyte growth factor from human leukocyte.", Biochemical Biophysical Research Communications, 172, 321-327 (1990)

### Summary of the Invention

### Problems to be Solved by the Invention

An object of the present invention is to provide a drug capable of well promoting granulation formation and neovascularization and being effective as a preventive or curative agent for skin ulcer.

### Means to Solve the Problems

The present inventors further continued to intensively study the invention described in the patent literature 1 and, as a result, found that, among HGFs, a human recombinant HGF wherein five amino acid residues are deleted in the first Kringle domain is excellent in the action of promoting granulation formation and neovascularization by exogenous supplementation. By utilizing such action of the human recombinant HGF, a drug excellent in wide tissue restoration can be provided. Especially, it was found out that the human recombinant HGF exerts the particularly excellent effect in preventing or treating a skin ulcer resulting from diabetes, phlebostasis, collagenosis or burn.

Further, the present inventors have continued to study, and completed the present invention.

That is, the present invention relates to:
(1) a composition for use in the promotion of the enclosure of skin ulcer by promoting the granulation formation and neovascularization , comprising a human recombinant HGF wherein five amino acid residues are deleted in the first Kringle domain, and/or a gene encoding said human recombinant HGF , wherein the human recombinant HGF in which five amino acid residues are deleted in the first Kringle domain is any one of the following;
   (a) a protein comprising an amino acid sequence described in SEQ ID NO: 1 of Sequence Listing;
   (b) a protein comprising an amino acid sequence in which one to several amino acid(s) is/are deleted, substituted or added in SEQ ID NO: 1 of Sequence Listing, and having the HGF activity; or
   (c) a protein encoded by a DNA comprising a nucleotide sequence which has 90% or more homology with a nucleotide sequence represented by SEQ ID No: 2 of the Sequence Listing, and having the HGF activity.
(2) The composition according to the above (1), wherein the gene encoding a human recombinant HGF in which five amino acid residues are deleted in the first Kringle domain is a gene comprising any one of the following DNAs:
   (a) a DNA comprising a nucleotide sequence described in SEQ ID NO: 2 of Sequence listing; or
   (b) a DNA comprising a nucleotide sequence in which one to several nucleotide(s) is/are deleted, substituted or added in SEQ ID NO: 2 of the Sequence Listing, and encoding a protein having the HGF activity.
(3) Use of a human recombinant HGF wherein five amino acid residues are deleted in the first Kringle domain and/or a gene encoding said human recombinant HGF, for preparing a drug for promoting the enclosure of skin ulcer by promoting neovascularization and granulation formation,
   wherein the human recombinant HGF in which five amino acid residues are deleted in the first Kringle domain is any one of the following:
   (a) a protein comprising an amino acid sequence described in SEQ ID NO: 1 of Sequence Listing;
   (b) a protein comprising an amino acid sequence in which one to several amino acid(s) is/are deleted, substituted or added in SEQ ID NO: 1 of Sequence Listing, and having the HGF activity; or
   (c) a protein encoded by a DNA comprising a nucleotide sequence which has 90% or more homology with a nucleotide sequence represented by SEQ ID NO: 2 of the Sequence Listing, and having the HGF activity.
(4) The use according to (3), wherein the gene encoding said human recombinant HGF in which five amino acid residues are deleted in the first Kringle domain is a gene comprising any one of the following DNAs:
   (a) a DNA comprising a nucleotide sequence described in SEQ ID NO: 2 of Sequence Listing; or
   (b) a DNA comprising a nucleotide sequence in which one to several nucleotide(s) is/are deleted, substituted or added in SEQ ID NO: 2 of Sequence Listing, and encoding a protein having the HGF activity.
(5) A sealing-type wound covering material, comprising a human recombinant HGF wherein five amino acid residues are deleted in the first Kringle domain, wherein the human recombinant HGF in which five amino acid residues are deleted in the first Kringle domain is any one of the following:
   (a) a protein comprising an amino acid sequence described in SEQ ID NO: 1 of Sequence Listing;
   (b) a protein comprising an amino acid sequence in which one to several amino acid(s) is/are deleted, substituted or added in SEQ ID NO: 1 of Sequence Listing, and having the HGF activity; or
   (c) a protein encoded by a DNA comprising a nucleotide sequence which has 90% or more homology with a nucleotide sequence represented by SEQ ID NO: 2 of the Sequence Listing, and having the HGF activity.
(6) A kit for promoting the enclosure of a skin ulcer by promoting the granulation formation and neovascularization, comprising a composition containing a human recombinant HGF wherein five amino acid residues are deleted in the first Kringle domain, and a sealing-type wound covering material which can absorb an exudate from an affected part of skin ulcer, wherein the human recombinant HGF in which five amino acid residues are deleted in the first Kringle domain is any one of the following:
   (a) a protein comprising an amino acid sequence described in SEQ ID NO: 1 of Sequence Listing;
   (b) a protein comprising an amino acid sequence in which one to several amino acid(s) is/are deleted, substituted or added in SEQ ID NO: 1 of Sequence Listing, and having the HGF activity; or
   (c) a protein encoded by a DNA comprising a nucleotide sequence which has 90% or more homology with a nucleotide sequence represented by SEQ ID NO: 2 of the Sequence Listing, and having the HGF activity.

### Effect of the Invention

The present invention provides a granulation formation promoting agent and a neovascularization promoting agent comprising a human recombinant HGF wherein five amino acid
wherein the human recombinant HGF in which five amino acid residues are deleted in the first Kringle domain is any one of the following:
(a) a protein comprising an amino acid sequence described in SEQ ID NO: 1 of Sequence Listing;
(b) a protein comprising an amino acid sequence in which one to several amino acid(s) is/are deleted, substituted or added in SEQ ID NO: 1 of Sequence Listing, and having the HGF activity; or
   a protein encoded by a DNA comprising a nucleotide sequence which has 90% or more homology with a nucleotide sequence represented by SEQ ID NO: 2 of the Sequence Listing, and having the HGF activity.
   (2) the use according to (1), wherein a gene encoding said human recombinant HGF in which five amino acid residues are deleted in the first Kringle domain is a gene comprising any one of the following DNAs:
      (a) a DNA comprising a nucleotide sequence described in SEQ ID NO: 2 of Sequence Listing;
      (b) a DNA comprising a nucleotide sequence in which one to several nucleotide(s) is/are deleted, substituted or added in SEQ ID NO: 2 of Sequence Listing, and encoding a protein having the HGF activity.
   (3) a sealing-type wound covering material, comprising a human recombinant HGF wherein five amino acid residues are deleted in the first Kringle domain,
   (4) a kit for promoting the enclosure of a skin ulcer by promoting the granulation formation and neovascularization, comprising a composition containing a human recombinant HGF wherein five amino acid residues are deleted in the first Kringle domain, and a sealing-type wound covering material which can absorb an exudate from an affected part of skin ulcer.

### Effect of the Invention

The present invention provides a granulation formation promoting agent and a neovascularization promoting agent comprising a human recombinant HGF wherein five amino acid residues are deleted in the first Kringle domain (hereinafter, simply referred to as "human recombinant HGF") or a gene encoding the human recombinant HGF. Such drug can be utilized in prevention or treatment of a skin ulcer. Further, since the human recombinant HGF is derived from a living body or originated from HGF derived from a living body, it is safe and has more little side effects when administered to a living body.

In addition, since the drug is such that, by using together with a sealing-type wound covering material, the affected part of the skin ulcer can be maintained under the wet environment, and a human recombinant HGF can be contacted with the skin ulcer surface in the hermetically sealed state, tissue restoration can be promoted.

### Brief Description of the Drawings

Fig. 1A shows a tissue image of a blood vessel stained in Example (3). Fig. 1B shows a blood vessel density of a human recombinant HGF-administered group and that of a physiological saline-administered group. In the figures, "physiological saline" indicates a physiological saline-administered group, and "HGF (10 µg)" indicates a human recombinant HGF-administered group. This is the same in the following figures.
Fig. 2A shows the extent of granulation tissue formation in a human recombinant HGF-administered group and a physiological saline-administered group. Fig. 2B shows a granulation area of a human recombinant HGF-administered group and that of a physiological saline-administered group.
Fig. 3 shows an ulcer enclosure rate (healing rate) of a human recombinant HGF-administered group and that of a physiological saline-administered group.

### Preferred Mode for Carrying Out the Invention

The human recombinant HGF used of the present invention is deleted in five amino acid residues among the first Kringle domain situated at 128^{th} to 206^{th} from the N-terminus in HGF comprising an amino acid sequence represented by SEQ ID NO: 3. Deleted five amino acid residues may be present continuously or discontinuously at 2 to 5 places, and preferably are present continuously. It is preferable that a human recombinant HGF used in the present invention comprising an amino acid sequence represented by SEQ ID NO: 1, or an amino acid sequence in which one to several amino acid(s) is/are deleted, substituted or added in said amino acid sequence. Especially, the human recombinant HGF comprising more preferably an amino acid sequence represented by SEQ ID NO: 1, or an amino acid sequence in which one to several amino acid(s) is/are substituted in the amino acid sequence, particularly preferably an amino acid sequence represented by SEQ ID NO: 1. It is preferable that a protein comprising an amino acid sequence in which one to several amino acid(s) is/are deleted, substituted or added in an amino acid sequence represented by SEQ ID NO: 1 has the HGF activity, that is, substantially the same quality of function as that of HGF, for example, the growth promoting activity on mature hepatocytes.

As a process for producing a human recombinant HGF used of the present invention using the gene recombination technique, the known process may be used, and the process described, for example, in "Biochemical Biophysical Research Communications, 172, 321-327 (1990)" (non-patent literature 17) is preferable.

Examples of a method of isolating and purifying the human recombinant HGF of the present invention from a living body include a method of treating a mature hepatocyte or platelet containing HGF at a relatively high concentration with thrombin; obtaining HGF secreted outside platelet; and purifying the HGF using ion exchange chromatography, affinity chromatography with heparin Sepharose or reversed phase high performance liquid chromatography. Usually, a human recombinant HGF in the present invention is isolated and purified using a human tissue or cell, but a tissue or a cell of a mammal other than a human such as rat, guinea pig, mouse, chicken, rabbit, pig, sheep, cow and monkey may be used for the isolation and purification of HGF as far as the isolated HGF satisfies the aforementioned requirements of the present invention.

The human recombinant HGF used of the present invention may have undergone various modifications known in the art. For example, a carboxyl group at the C-terminus of a human recombinant HGF used in the present invention may be amidated, esterified or ionized. Specifically, a carboxyl group (-COOH) at the C-terminus may be substituted with carboxylate (-COO⁻), amido (-CONH₂) or ester (-COOR). Herein, examples of the substituent R in an ester include a C₁₋₆ alkyl group such as methyl, ethyl, n-propyl, isopropyl and n-butyl; a C₃₋₈ cycloalkyl group such as cyclopentyl and cyclohexyl; a C₆₋₁₂ aryl group such as phenyl and α-naphthyl; a phenyl-C₁₋₂ alkyl group such as benzyl and phenethyl, and a C₇₋₁₄ aralkyl group such as an α-naphthyl-C₁₋₂ alkyl group such as α-naphthylmethyl; and a pyvaloyloxymethyl group; and the like. Further, when a human recombinant HGF used in the present invention has a carboxyl group at a position other than the C-terminus, such carboxyl group may be amidated, esterified or ionized as described above.

The human recombinant HGF used in the present invention includes an HGF in which an amino group of the methionine residue at the N-terminus is protected with a protecting group (e.g. C₁₋₆ acyl group such as formyl and C₂₋₆ alkanoyl group including acetyl) ; an HGF in which the glutamyl group produced by cleavage of the N-terminal side in a living body is converted into pyroglutamic acid; an HGF in which the substituent (e.g. -OH, -SH, amino group, imidazole group, indole group, guanidino group etc.) of the amino acid side chain is protected with a suitable protecting group (e.g. C₁₋₆ acyl group such as formyl and C₂₋₆ alkanoyl group including acetyl); and a composite protein such as the so-called glycoprotein with a sugar chain bound thereto. Especially, as a human recombinant HGF used in the present invention, an HGF with a sugar chain bound thereto is desirable, and examples of a sugar to be bound include fucose, galactose, mannose, and N-acetylglucosamine.

The human recombinant HGF used in the present invention may form a pharmacologically acceptable salt. When the human recombinant HGF of the present invention exhibits basicity, examples of the pharmacologically acceptable salt include salts with an inorganic acid (e.g. hydrochloric acid, phosphoric acid, hydrobromic acid, sulfuric acid), and salts with an organic acid (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid). When the human recombinant HGF in the present invention exhibits acidity, examples of the pharmacologically acceptable salt include salts with an inorganic base (e.g. alkali metal salt such as sodium salt and potassium salt; alkaline earth metal salt such as calcium salt and magnesium salt; aluminum salt; and ammonium salt), and salts with an organic base (e.g. trimethylamine, triethylamine, pyridine, picoline, ethanolamine, diethanolamine, triethanolamine, dicyclohexylamine, and N,N'-dibenzylethylenediamine etc.).

The gene encoding a human recombinant HGF used in the present invention is not particularly limited as far as it is a gene encoding the aforementioned human recombinant HGF, but a gene comprising any one of the following DNAs (a) a DNA comprising a nucleotide sequence represented by SEQ ID NO: 2, and (b) a DNA comprising a nucleotide sequence in which one to several nucleotide(s) is/are deleted, substituted or added in SEQ ID NO: 2, and encodes a protein having the HGF activity. In the present invention, it is particularly preferable to use a DNA containing or comprising a nucleotide sequence represented by SEQ ID NO: 2.

The DNA which hybridizes with a DNA comprising a nucleotide sequence represented by SEQ ID NO: 2 means a DNA obtained by, for example, using a colony hybridization method, a plague hybridization method or a Southern blot hybridization method, with use of the aforementioned DNA as a probe. Specifically, examples include a DNA identified by performing hybridization at about 65°C in the presence of about 0.7 to 1.0 M of sodium chloride using a filter with a colony or plaque-derived DNA immobilized thereon, and washing the filter under the condition of about 65°C using an SSC solution having about 0.1 to 2-fold concentration (composition of SSC solution having 1-fold concentration comprises 150 mM sodium chloride and 15 mM sodium citrate).

Specific examples of the DNA which hybridizes with a DNA comprising a nucleotide sequence represented by SEQ ID NO: 2 include a DNA comprising a nucleotide sequence having about 70% or more, preferably about 80% or more, more preferably about 90% or more, most preferably about 95% or more homology with a nucleotide sequence represented by SEQ ID NO: 2. Hybridization can be performed according to the known method, for example, the method described in Molecular Cloning, A Laboratory Manual, Third Edition (J. Sambrook et al. , Cold Spring Harbor Lab. Press, 2001 (hereinafter, abbreviated as Molecular Cloning Third Edition). In addition, when a commercially available library is used, hybridization can be performed according to the method described in an attached written instruction.

The gene encoding a human recombinant HGF used in the present invention may be any one of a genomic DNA, a genomic DNA library, a cDNA derived from the aforementioned cell/tissue, a cDNA library derived from the aforementioned cell/tissue, and a synthetic DNA. The vector used in the library may be any one of bacteriophages, plasmids, cosmids and phagemids. The DNA encoding a human recombinant HGF may be also obtained by amplification directly by a RT-PCR method using a total RNA or a mRNA fraction prepared from the aforementioned cell/tissue. In addition, an RNA can be used as far as it can express a human recombinant HGF in the present invention by a reverse transcriptase. The RNA can be also obtained by the known means .

The gene encoding a human recombinant HGF used in the present invention can be obtained by the known method, and it is preferable to used a method described, for example, in the aforementioned "Biochemical Biophysical Research Communications, 172, 321-327 (1990)" (non-patent literature 17). Further, the resulting gene may be subjected to the known treatment. For example, substitution of a nucleotide sequence of a DNA can be performed according to the known method such as the ODA-LA PCR method, the gapped duplex method, the Kunkel method and the like or a similar method using PCR or the known kit, for example, Mutan ™-super Express Km (TAKARA SHUZO), Mutan ™-K (TAKARA SHUZO) or the like. Alternatively, a gene may be digested with a restriction enzyme, or a linker may be added. Further, a translation initiation codon (ATG) and a translation termination codon (TAA, TGA or TAG) may be added using a suitable synthetic DNA adapter.

The gene encoding a human recombinant HGF in the present invention may be modified in order to enhance its stability in a cell or reduce the toxicity if it has toxicity. Such modification includes a modification by the method described, for example, in J. Kawakami et al., Pharm Tech Japan, Vol.8, p247 (1992) ; Vol.8, p395 (1992) ; S. T. Crooke et al. ed. , Antisense Research and Applications, CRC Press (1993). Further examples include a modification by addition of other substances other than adenine, thymidine , guanine and cytosine . Examples of such other substance include hydrophobic substances such as a sugar; an acid or a base; a polycationic compound such as polylysine which serves to neutralize a charge of a phosphate group skeleton; and a lipid. As a lipid to be preferably added, a lipid contributing to improvement in interaction with a cell membrane or increase in uptake of a nucleic acid is preferable, and specific examples include cholesterol, a derivative thereof (e.g. cholesteryl chloroformate or cholic acid) and a phospholipid. The other substances may be attached to the 3'-terminus or the 5'-terminus of nucleic acids, or may be attached via a base, a sugar, or an intramolecular nucleoside linkage. Additional examples of the modification of a gene encoding a human recombinant HGF in the present invention include chemical modification of the end of the gene. Examples of a modifying group for the end include a group for capping which is specifically disposed at the 3'-terminus or the 5' -terminus of nucleic acids and is for arresting degradation with a nuclease such as exonuclease and RNase. Examples of the group for capping include, though not limited to, a protecting group for the hydroxy group known in the art including glycols such as polyethylene glycol and tetraethylene glycol.

The gene encoding a human recombinant HGF of the present invention may be used in the formulation as it is, or may be used in the form of being inserted into an expression vector. The expression vector is enough if it can express a human recombinant HGF of the present invention, and examples include a vector in which a DNA fragment encoding a human recombinant HGF of the present invention is ligated downstream of a suitable promoter.

As the expression vector, there are used a plasmid derived from Escherichia coli (e.g. pCR4, pCR2.1, pBR322, pBR325, pUC12, pUC13), a plasmid derived from Bacillus subtilis (e.g. pUB110, pTP5, pC194), a plasmid derived from yeast (e.g. pSH19, pSH15), bacteriophage such as λ phage, virus such as retrovirus, adeno-associated virus (AAV), adenovirus, lentivirus, vaccinia virus, baculovirus, poxvirus, herpes virus, herpes simplex virus , lentivirus (HIV), sendai virus, Epstein-Barr virus (EBV), vaccinia virus, poliovirus, sindbis virus, SV40 and the like and, additionally, pA1-11,pXT1,pRc/CMV,pRc/RSV, and pcDNAI/Neo. Especially, a virus is preferable, and it is preferable to use adeno-associated virus (AAV), adenovirus, retrovirus, poxvirus, herpesvirus, herpes simplex virus, lentivirus (HIV), sendai virus, Epstein-Barr virus (EBV), vaccinia virus, poliovirus, sindbis virus, SV40 or the like. Furthermore, it is more preferable to use adeno-associated virus (AAV) or adenovirus. There are various serotypes in adenovirus, but in the present invention, it is preferable to use human adenovirus type 2 or type 5.

In addition, when an expression vector is not introduced into a host cell as described later, but is in vivo introduced as a naked vector into a living body, as an expression vector to be used, a plasmid such as pCAGGS (Gene, 108, p193-200 (1991)), pBK-CMV, pcDNA3.1, pZeoSV (Invitrogen, Stratagene) and the like is preferable.

As the promoter, any promoter may be used as far as it is an appropriate promoter depending on a host used in gene expression. For example, when an animal cell is used as a host, examples of such promoters include an SRα promoter, an SV40 promoter, a LTR promoter, a CMV promoter, and an HSV-TK promoter. Among them, it is preferable to use a CMV promoter or an SRα promoter. When the host is a bacterium of the genus Escherichia, a trp promoter, a lac promoter, a recA promoter, a λP_{L}, promoter and a lpp promoter are preferable. When a host is a bacterium of the genus Bacillus, preferred are an SPO1 promoter, an SPO2 promoter and a penP promoter. When the host is yeast, preferred are a PHO5 promoter, a PGK promoter, a GAP promoter and an ADH promoter. When the host is an insect cell, preferred are a polyhedrin promoter and a P10 promoter.

The expression vector may have an enhancer, a splicing signal, a polyA addition signal, a selectable marker or an SV40 replication origin, if desired, in addition to a gene encoding a human recombinant HGF of the present invention and a promoter. Examples of the selectable marker include a dihydrofolate reductase (hereinafter, abbreviated as dhfr in some cases) gene (methotrexate (MTX) resistant), an ampicillin resistant gene, a neomycin resistant gene (G418 resistant) and the like. In particular, when a dhfr gene-deficient Chinese hamster cell CHO is used and a dhfr gene is used as a selectable marker, an objective gene can be selected also by a medium containing no thymidine. In addition, as a signal sequence, when the host is a bacterium of the genus Escherichia, a PhoA· signal sequence or an Omba signal sequence can be utilized. When the host is a bacterium of the genus Bacillus, an α-amylase signal sequence or a subtilisin signal sequence can be utilized. When the host is yeast, an MFα signal sequence or an SUC2 signal sequence can be utilized. When the host is an animal cell, an insulin signal sequence, an α-interferon signal sequence or an antibody molecule signal sequence can be utilized.

The thus constructed expression vector containing a gene encoding a human recombinant HGF of the present invention is further introduced into a host, and can be used in the form of a transformant in its formulation. By administration of a transformant, a human recombinant HGF of the present invention is produced in a cell, in a cell membrane or outside a cell of a transformant in an administered living body, whereby the human recombinant HGF of the present invention can be effectively administered to a living body.

As a host into which the expression vector is introduced, there can be used, for example, a bacterium of the genus Escherichia, a bacterium of the genus Bacillus, bifido-bacterium, lactic acid bacterium, yeast, an insect cell, an insect, and an animal cell. Examples of the bacterium of the genus Escherichia include Escherichia coli K12 DH1 (Proc. Natl. Acad. Sci. USA,vol 60,160 (1968)), JM103(NucleicAcids Research, vol 9, 309 (1981)), JA221 (Journal of Molecular Biology, vol 120, 517 (1978)), HB101 (Journal of Molecular Biology, vol 41, 459 (1969)), C600 (Genetics, vol 39, 440 (1954)), DH5α (Inoue, H. , Nojima, H. andOkayama, H., Gene, 96, 23-28 (1990)), DH10B (Proc. Natl. Acad. Sci. USA, vol 87, 4645-4649 (1990)) and the like. Examples of the bacterium of the genus Bacillus include Bacillus subtilis MI114 (Gene, vol 24, 255 (1983), Journal of Biochemistry, vol 95,87 (1984)) and the like. Examples of the bifido-bacterium include Bifidobacterium longum, Bifidobacterium bifidum, Bifidobacterium breve and the like. Examples of the lactic acid bacterium include the genus Lactobacillus, the genus Streptoccoccus, the genus Leuconostoc, the genus Pediococcus and the like. Examples of the yeast include Saccharomyces cerevisiae AH22, AH22R⁻, NA87-11A, DKD-5D, 20B-12, Schizosaccharomyces pombe NCYC1913, NCYC2036, Pichia pastoris and the like.

Examples of the insect cell include, when a virus is AcNPV, an established cell derived from a larva of cabbage armyworm (Spodoptera frugiperda cell; Sf cell), an MG1 cell derived from a midgut of Trichoplusia ni, a High Five™ cell derived from an egg of Trichoplusia ni, a cell derived from Mamestrabrassicae, and a cell derived from Estigmena acrea and, when a virus is BmNPV, an established cell derived from silkworm (Bombyx mori N; BmN cell). As the Sf cell, for example, an Sf9 cell (ATCC CRL1711), an Sf21 cell (all, Vaughn, J.L. et. al., In Vivo, 13, p213-217 (1977)) and the like are used. Example of the insect include a larva of a silkworm (Maeda et al, Nature, 315, 592 (1985)).

Examples of the animal cell include a monkey cell COS-7, Vero, Chinese hamster cell (CHO) (hereinafter, abbreviated as CHO cell), a dhfr gene-deficient Chinese hamster cell CHO (hereinafter, abbreviated as CHO(dhfr⁻)), a mouse L cell, mouse AtT-20, a mouse myeloma cell, rat GH3, a human FL cell and the like.

For transforming a bacterium of the genus Escherichia, transformation can be performed according to the method described, for example, in "Proc. Natl. Acad. Sci. USA, 69, p2110 (1972)" or "Gene, 17, p107 (1982)". For transforming a bacterium of the genus Bacillus, transformation can be performed according to the method described, for example, in "Molecular & General Genetics, 168, p111 (1979)". For transforming yeast, transformation can be performed according to the method described, for example, in "Methods in Enzymology, vol 194, p182-187 (1991) " , "Proc. Natl. Acad. Sci. USA, 75, p1929 (1978)" or the like. For transforming an insect cell or an insect, transformation can be performed according to the method described, for example, in "Bio/Technology, 6, p47-55 (1988)" or the like. For transforming an animal cell, transformation can be performed according to the method described, for example, in "Cell Technology, Separate Volume 8, New Cell Technology Experimental Protocol, p263-267 (1995) (issued by Shujunsha)", "Virology, vol. 52, p456 (1973)" or the like.

By culturing the thus obtained transformant, a raw material for drugs relating to the present invention can be obtained at a large amount. Upon culturing of a transformant where a host is a bacterium of the genus Escherichia or a bacterium of the genus Bacillus, a liquid medium is appropriate as a medium used for culturing, and a carbon source, a nitrogen source, an inorganic substance and others necessary for the growth of the transformant are contained therein. Examples of the carbon source include glucose, dextrin, soluble starch and sucrose, examples of the nitrogen source include inorganic or organic substances such as ammonium salts, nitrates, corn steep liquor, peptone, casein, meat extract, soybean cake and potato extract solution, and examples of the inorganic substance include calcium chloride, sodium dihydrogen phosphate and magnesium chloride. Further, yeast extract, vitamins, and growth promoting factor may be added. A pH of the medium is desirably about 5 to 8.

As a medium for culturing a bacterium of the genus Escherichia, for example, an M9 medium (Miller, Journal of Experiments in Molecular Genetics, p431-433, Cold Spring Harbor Laboratory, New York (1972)) containing glucose and casamino acid is preferable. Herein, if necessary, in order to effectively act on a promoter, a drug such as 3β-indolylacrylic acid can be added. When the host is a bacterium of the genus Escherichia, culturing is usually performed at about 15 to 43°C for about 3 to 24 hours, and aeration or stirring may be carried out, if necessary. When the host is a bacterium of the genus Bacillus , culturing is usually performed at 30 to 40°C for about 6 to 24 hours, and aeration or stirring may be conducted, if necessary. Upon culturing of a transformant where the host is yeast, examples of the medium include a Burkholder minimum medium (Bostian, K. L. et al. , Proc. Natl. Acad. Sci. USA, 77, p4505 (1980)), an SD medium (Bitter, G. A. et al., Proc. Natl. Acad. Sci. USA, 81, p5330 (1984)) containing 0.5% casamino acid, and the like. It is preferable to adjust the pH of the medium to about 5 to 8. Culturing is usually performed at about 20°C to 35°C for about 24 to 72 hours, and aeration or stirring is performed, if necessary.

Upon culturing of a transformant where the host is an insect cell or an insect, as a medium, Grace's Insect Medium (Grace, T.C.C., Nature, 195, p788 (1962)) to which an additive such as immobilized 10% bovine serum and the like is appropriately added, is used. It is preferable to adjust the pH of the medium to about 6.2 to 6.4. The culturing is usually performed at about 27°C for about 3 to 5 days, and aeration or stirring is added, if necessary. Upon culturing of a transformant where the host is an animal cell, as a medium, for example, an MEMmedium (Science, 122, p501 (1952)) containing about 5 to 20% fetal bovine serum, a DMEM medium (Virology, 8, p396 (1959)), an RPMI 1640 medium (The Journal of the American Medical Association, 199, p519 (1967)), a 199 medium (Proceeding of the Society for the Biological Medicine, 73, p1 (1950)) and the like are used. The pH is preferably about 6 to 8. The culturing is usually performed at about 30°C to 40°C for about 15 to 60 hours and aeration or stirring is performed, if necessary.

In the present invention, upon formulation into a preparation, a gene encoding a human recombinant HGF of the present invention or an expression vector containing the gene may be used in a form in which it is encapsulated in an artificial vector such as a liposome, a microcapsule, a microsphere, a cytofectin, a DNA-protein complex and a biopolymer. Especially, it is preferable to encapsulate it into a liposome or a microcapsule.

Herein, a liposome is a closed vesicle composed of a lipid bilayer membrane having an aqueous layer in the interior thereof, and it is known that its lipid bimolecular membrane structure is extremely similar to a living body membrane. Examples of a phospholipid which is used in preparing a liposome include acidic phospholipid such as phosphatidylcholine such as lecithin, lysolecithin and the like, phosphatidylserine, phosphatidylglycerol and the like, and sphingophospholipid such as phosphatidylethanolamine, sphingomyelin and the like. Alternatively, cholesterol or the like may be added. A liposome can be prepared according to the method known per se. As a liposome, a membrane-fused liposome, a HVJ-membrane-fused liposome (Kaneda. Y et al. , Biol. Chem, 264, p12126-12129 (1989), Kato. K et al., Biol. Chem, 266, p3361-3364 (1991), Tomita. N et al., Biochem. Biophys. Res., 186, p129-134 (1992), Tomita. N et al., Circ. Res., 73, p898-905 (1993)), a cationic liposome (Japanese Patent Application National Publication (Laid Open) No. 2000-510151, Japanese Patent Application National Publication (Laid Open) No. 2000-516630) and the like are known. In the present invention, it is preferable to use an HVJ-membrane-fused liposome fused with Sendai virus (HVJ). When a glycoprotein of HVJ is incorporated into a surface of a liposome, or covalently bound thereto, and polyethylene glycol is added, an efficacy of introduction of a gene into a cell is increased. A microcapsule is a film-coated particle, and examples include a microcapsule composed of a particle coated with a coating material consisting of a mixture of a film forming polymer derivative, a hydrophobic plasticizer, a surface activating agent or/and a lubricant nitrogen-containing polymer.

The neovascularization promoting agent and the granulation formation-promoting agent (hereinafter, these are collectively referred to as "present drug") of the present invention contain the afore mentioned human recombinant HGF of the present invention or gene encoding a human recombinant HGF of the present invention as an active ingredient. As the drug of the present invention, there are also included (a) a neovascularization promoting agent or a granulation formation-promoting agent comprising an expression vector containing a gene encoding a human recombinant HGF of the present invention, (b) a neovascularization promoting agent or a granulation formation-promoting agent containing a transformant comprising an expression vector containing a gene encoding a human recombinant HGF of the present invention.

The drug of the present invention is prepared by formulating the aforementioned active ingredient usually together with an additive for preparations used in the art according to a conventional method. The dosage form of the drug of the present invention is not particularly limited, but examples include tablets, pills, capsules, powders, granules, suppositories, injections, pastes, ointments, creams, gells, gell-like creams, lotions, emulsions, suspensions, cataplasms, plasters, liniments, aerosols, syrups, buccals, eye drops and nasal drops. The tablets may be coated tablets such as sugar-coated tablets, gelatin-encapsulated tablets, enteric coated tablets and film-coated tablets, bilayered tablets or multilayered tablets.

The aforementioned pharmaceutical preparation can be prepared according to the method which is well-known per se or conventional in the field of pharmacy.

For preparing solid preparations such as tablets, pills, capsules, powders and granules, for example, excipients, binders, disintegrating agents, surfactants or lubricants can be used as a formulation additive. Examples of the excipients include sugars such as lactose, white sugar and glucose; starches such as starch; and crystalline cellulose and the like. Examples of the binders include sugars or sugar alcohols such as glucose and maltitol; polysaccharides such as starch; natural polymers such as gelatin; cellulose derivatives such as methylcellulose and carboxymethylcellulose; synthetic polymer compounds such as polyvinylpyrrolidone and the like. Examples of the binders include sugars or sugar alcohols such as glucose and maltitol; polysaccharides such as starch; natural polymers such as gelatin; cellulose derivatives such as methylcellulose and carboxymethylcellulose; synthetic polymer compounds such as polyvinylpyrrolidone and the like. Examples of the disintegrating agents include starch, sodium alginate, corn starch, hydroxypropylstarch, polyvinylpyrrolidone and sodium croscarmellose. Examples of lubricants include stearic acid salt, talc, boric acid powder and polyethylene glycol. Example of the surfactants include fatty acid ester and the like.

When the drug of the present invention has a dosage form of suppositories, it can be prepared by incorporating the aforementioned active ingredient and, if desired, formulation additives such as local anesthetics, anti-histamines, local astringents, sulfa drugs, antibiotics, wound healing agents, surfactants, vitamins, crude drug extracts, bile acids, antiseptics, excipients, absorption promoting agents and amino acids into a lipophilic base, a water-soluble base or an emulsifiable base.

When the drug of the present invention has a dosage form of injections, the drug of the present invention can be prepared by incorporating the aforementioned active ingredient and, if desired, formulation additives such as solubilizers, buffers and soothing agents in a solvent such as a water-soluble solvent and a non-water-soluble solvent. The injections are preferably sterilized and isotonic with blood, and may contain sodium chloride, glucose or glycerin in order to be isotonic with blood. Further, if desired, the pharmaceutical preparation may contain coloring agents, preservatives, perfumes, flavors, sweeteners and the like.

When the drug of the present invention has a dosage form of ointments, it can be prepared by incorporating the aforementioned active ingredient and, if desired, formulation additives such as emulsifiers such as anionic or non-ionic surfactants, and preservatives such as paraoxybenzoic acid esters into a base such as an oleaginous base such as vaseline, liquid paraffin, silicone and vegetable oil; an emulsifiable base such as hydrophilic vaseline and purified lanolin; a water-soluble base such as macrogol.

When the drug of the present invention has a dosage form of gels, it can be prepared by incorporating the aforementioned active ingredients and, if desired, formulation additives such as lower alcohols, neutralizing agents, surfactants and absorption promoting agents into a base obtained by adding a gelling agent (e.g. carboxyvinyl polymer, hydroxyethylcellulose, hydroxypropylcellulose, methylcellulose, ethylcellulose, carboxymethylcellulose and alginic acid propylene glycol ester, etc.) to water.

When the drug of the present invention has a dosage form of creams , it can be prepared by incorporating the aforementioned active ingredient and, if desired, formulation additives such as emulsifiers, antiseptics, absorption promoting agents, rash preventing agents into a base containing, for example, higher fatty acid esters (e. g. myristic acid ester, palmitic acid ester, diethyl sebacate, hexyl laurate, cetyl isooctanate, etc.), lower alcohols (e.g. ethanol, isopropanol, etc.), carbohydrates(e.g. liquid paraffin, squalane, etc.), polyhydric alcohols (e.g. propylene glycol, 1,3 butylene glycol, etc.) or higher alcohols (e.g. 2-hexyldeconal, cetanol, 2-octyldodecanol, etc.).

In addition, in order to obtain gel like creams having a nature between creams and gels, a gelling agent and a neutralizing agent may be added to the aforementioned creams.

When the drug of the present invention has a dosage form of liquid for external use, it can be prepared by incorporating the aforementioned active ingredient and, if necessary, formulation additives such as buffers , stabilizers, antiseptics, pH adjusting agents, solvents, solubilizers, flavors, gelling agents, corrigents and refreshing agents into a solvent. Examples of the solvent include glycerin, propylene glycol, ethanol, isopropanol, butylenes glycol, water, sorbitol, mannitol, xylitol, glucose, ε-aminocaproic acid, glycine, glutamic acid salt, sodium hyaluronate, polyethylene glycols, carboxyvinyl polymers, higher alcohols (e.g. cetanol, stearyl alcohol, etc.), fatty acid esters (e.g. medium-sized fatty acid esters, isopropyl myristate, etc.), higher fatty acids (e.g. stearic acid, etc.), squalane, liquid paraffin, white vaseline and purified lanolin.

Herein, examples of the liquid for external use include liquid preparations which are externally used for washing, injection, compress, inhalation, spraying, enema administration, coating, drug bath, toilet, disinfection, eye drop, eye washing, ear drop or nasal drop.

The aerosol can be prepared by using the liquid for external use of the present invention usually together with a propellant. Examples of the propellant include dimethyl ether, liquefied petroleum gas, N₂ gas, nitrous oxide gas, CO₂ gas, alternative tofreongas, and the like. The compressed air may be used without use of a propellant. Alternatively, a mixture thereof may be used.

For the drug of the present invention, an administration method may be appropriately selected from various administration methods such as oral administration, parenteral administration and local administration, depending on a dosage form thereof. The drug of the present invention may be implanted subcutaneously or intramuscularly.

The gene encoding a human recombinant HGF of the present invention may be directly administered to a living body, without formulation as it is, as described above, or together with an auxiliary agent for promoting ingestion. Such administration method may be performed according to the known method and, for example, there are an in vivo method of directly introducing a DNA into a body, and an ex vivo method of taking a certain kind of a cell out of a human to be administered, introducing a gene encoding a human recombinant HGF into the cell, and returning a transformed cell into a body (Nikkei Science, April, 20-45 (1994), The Pharmaceuticals Monthly, 36, 23-48 (1994), Experimental Medicine, Extra Edition, 12, 15 (1994)). In each of the methods, as a method of introducing a gene encoding a human recombinant HGF of the present invention into a cell, there are a gene transfer method of introducing into a cell a gene contained in an expression vector such as an adeno-associated virus, an adenovirus vector, a retrovirus vector and the like as described above, transfection, electroporation, microinjection, transduction, cell fusion, DEAE-dextran method, calcium phosphate precipitation, a method of introducing into a cell a DNA together with a carrier (metal particle, etc.) with a gene gun (Wu et al., J. Biol. Chem. 267, 963-967 (1992), Wu et al., J. Biol. Chem. 263, 14621-14624, (1988), Proc. Natl. Acad. Sci., USA, 88, 2726-2730 (1991)), and the like. When a liposome or the like is used, examples include a liposome method, an HVJ-liposome method, a cationic liposome method, a lipofectin method, a lipofectamine method and the like. Especially, from a viewpoint of an introduction efficacy, a gene transfer method using an adenovirus vector or a retrovirus vector is desirable.

In addition, the neovascularization promoting agent and the granulation formation-promoting agent of the present invention are used as an external preparation, they are preferably used together with a sealing-type wound covering agent. As the sealing-type wound covering material, a covering material which provides the wet environment at a wound part, and can permeate oxygen and water steam without permeating a liquid and bacteria is preferable. Preferable examples of such sealing- type wound covering material include dressing materials such as a hydrocolloid dressing material (e.g. DuoActive (Convatec), Comphile (Coloplast), Tegasorb (3M), Absocure (Nitto Medical Corporation)), a kitchen dressing material, an alginate dressing material (e.g. Cartstat (Convatec), Sorbsan (Alcare Co., Ltd), Algodam (Medion Inc.), Kurabio AG (Kuraray Co., Ltd)), ahydrogendressingmaterial (e.g. Jelley-palm (Taketora), New Jail (Johnson&Johnson), Intrasite (Smith & Nephew), Granugel (Convatec), Clearsite (Nippon Sigmax Co. , Ltd)), a polyurethane dressing material (e.g. Tegadam (3M), Opsite Wound (Smith & Nephew), IV3000 (Smith & Nephew), Bioclusive (Johnson & Johnson)), a hydropolymer dressing material (e.g. Tieral (Johnson & Johnson)), a hydrofiber dressing material, and a polyurethane foam (e.g. Hydrosite (Smith & Nephew)). These dressing materials may be used alone or in combination of one or two or more thereof. Alternatively, a dressing material may be a form such as a pressure-sensitive adhesive sheet and a film for skin application.

As a form of combination use of the external preparation of the present invention and a sealing-type wound covering material, the external preparation may be contained in a sealing-type wound covering material, or after the external preparation of the present invention may be coated, a damaged site is covered with a sealing-type wound covering material, or after a damaged site is covered with a sealing-type wound covering material, the external preparation of the present invention may be injected with, for example, an injector.

When the external preparation of the present invention is contained in a sealing-type wound covering material, it is usually preferable that the external preparation of the present invention is contained in an absorber layer (e.g. hydrogel layer, etc.) of a sealing-type wound covering material. In this case, it is suitable that a human recombinant HGF is added in an amount of 0.01% by mass to 5% by mass, preferably 0.1% by mass to 3% by mass based on a dry weight of an absorber layer as a standard. By containing a human recombinant HGF in an absorber layer, for example, as an absorber layer is wetted with an exudate, the release level of a human recombinant HGF can be enhanced. In addition, when a damaged site is covered with a sealing type wound covering material after the external preparation of the present invention is applied, there is an advantage that the external preparation of the present invention is not leaked outside. Alternatively, the external preparation of the present invention can be administered without exposing the wound surface to the open air by injecting the external preparation of the present invention into a wound surface or an absorber layer of a wound covering material with, for example, an injector after a damaged site is covered with a sealing-type wound covering material.

The dose and an administration frequency of the drug of the present invention are not particularly limited, but can be appropriately selected depending on various conditions such as the kind of pathema to be treated, age and body weight of patients, symptom and severity of diseases, and the like. For example, in the case of intravenous administration, the dose as an amount of a human recombinant HGF is about 250 to 1000 µg/Kg/day, preferably about 300 to 800 µg/Kg/day, particularly preferably about 300 to 550 µg/Kg/day, and the dose as an amount of a gene encoding a human recombinant HGF is about 0.2 to 40, 000 µg/Kg/day, preferably about 2 to 2,000 µg/Kg/day. In the case of topical application of the external medicine, the dose of an active ingredient is about 0.001 to 30 mg, preferably about 0.01 to 3 mg.

The drug of the present invention can be used as a neovascularization promoting agent or a granulation formation-promoting agent. Furthermore, by utilizing the neovascularization promoting action or the granulation formation-promoting action, the drug of the present invention can be also used as a hair restorer or a cosmetic, in addition to medicinal use in digestive ulcer therapy, anti-cancer agent, skin suturing after operation, and corner operation including preventive therapy for skin ulcer.

### Exemplary Embodiments

The present invention will be specifically explained below by way of Examples, but it goes without saying that the present invention is not limited to them.

### (1) Preparation of human recombinant HGF of the present invention

According to the method described in "Seki, T., Ihara, I., Sugimura, A., Shimonishi, M., Nishizawa, T., Asami, O., Hagiya, M., Nakamura, T. and Shimizu, S., "Isolation and expression of cDNA for different forms of hepatocyte growth factor from human leukocyte.", Biochemical Biophysical Research Communications, 172, 321-327 (1990)", a human recombinant HGF wherein five amino acid residues are deleted in the first Kringle domain was purified from the culture supernatant of CHO cells with an expression vector introduced therein. It was found that the purification degree of the human recombinant HGF was 98 % or more by the protein staining method after SDS gel electrophoresis. An amino acid sequence of the human recombinant HGF wherein five amino acid residues are deleted in the first Kringle domain is shown in SEQ ID NO: 1.

### (2) Production of skin ulcer model, and topical administration of human recombinant HGF

Mutated mice (female of C57BL/KsJ-db/db, 10 weeks of age) which spontaneously develop diabetes were employed in an experiment. A skin entire layer-defective ulcer model having a diameter of 6 mm was made on two places of the back of each mouse using a punch biopsy (equipment for collecting skin biopsy) . Thereafter, the affected part of ulcer was covered with a transparent semipermeable covering material (BIOCLUSIVE, Johnson & Johnson MEDICAL) . The day of entire layer deficiency treatment was set to 0^{th} day, and the solution of the human recombinant HGF prepared in the above (1) or a physiological saline (25 µl) as a comparative example was administered to the ulcer part from above a covering material using a 2 7 gauge injector, for consecutive days from immediately after producing the ulcer model. The dose of the human recombinant HGF is 10 µg/ulcer/day. On the 7^{th} day, 14^{th} day, 21^{st} day and 28^{th} day after producing an ulcer model, the mice were analyzed for the affected part of the ulcer.

### (3) Histlogical analysis and neovascularization analysis

In the case where tissue staining of blood vessel was performed, a 70% ethanol-fixed paraffin-embedded section was treated with 0.1% trypsin at room temperature for 10 minutes, washed with PBS (phosphate-buffered physiological saline), and treated with PBS containing 3% hydrogen peroxide for 30 minutes . Then, the section was incubated with an anti-CD31/PECAM-1 antibody (50-fold diluted antibody of PharMingen) at room temperature for 2 hours, and incubated with a biotin-labeled horseradish peroxidase-bound anti-rat IgG antibody (200-fold diluted antibody of DAKO) for 30 minutes. The enzyme antibody reaction was detected in a substrate solution containing diaminobenzidine and hydrogen peroxide and, after detection, the cell nucleus was stained with hematoxylin. A blood vessel density was quantitated by observation of the section with a microscope (100-fold visual field).

### (4) Analysis of granulation tissue

In the case where a granulation tissue was measured, a tissue section stained with hematoxylin and eosin was used. An image of the tissue section prepared so as to be vertical to the surface of the affected part of ulcer was inputted into a computer, a granulation tissue was traced, and an area of a granulation tissue was quantitated with an NIH image analyzing software.

### (5) Determination of enclosure (healing) rate at ulcer part

In order to measure enclosure (healing) of an ulcer part, an ulcer part section edge was traced on a slide glass so as to be inputted into a computer with a CCD camera, and an area of the ulcer part was quantitated by image analysis using an NIH image analyzing software. The enclosure rate 0% at an ulcer part corresponds to a state where no enclosure of an ulcer part is recognized, and the rate 100% corresponds to completion of enclosure of an ulcer part.

The results of the experiment are shown below.

### (Result-1) Spur of neovascularization with human recombinant HGF

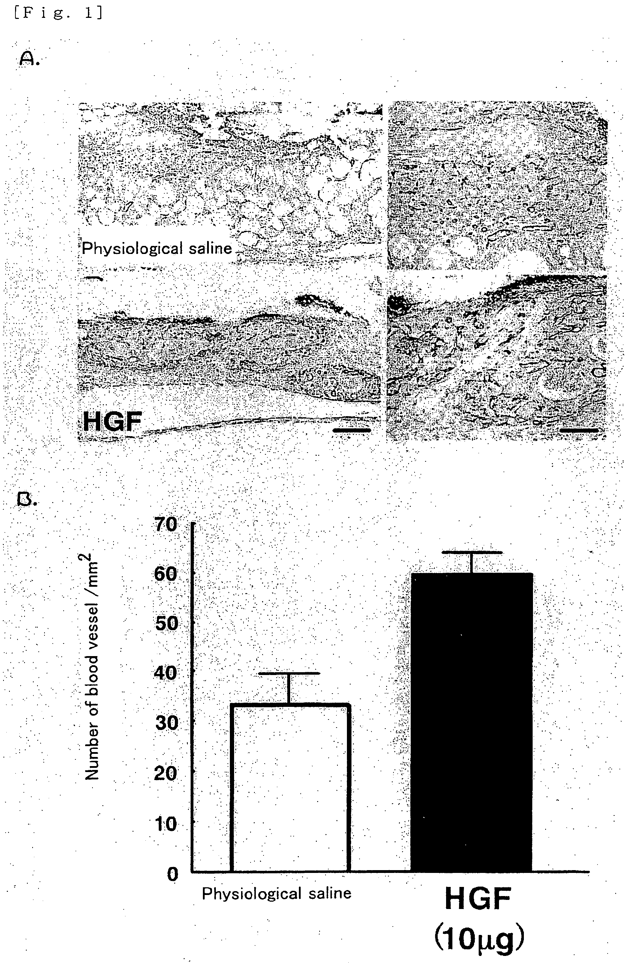

Since neovascularization is responsible for an important role in tissue restoration including healing of skin ulcer, the effect of a human recombinant HGF on neovascularization was analyzed. Using a tissue section 7 days after producing a skin ulcer model, a blood vessel was detected by immunostaining (Fig. 1A shows a tissue image of stained blood vessels) . As a result, a blood vessel density was 33.4±6.0/mm² in a control (physiological saline-administered group), whereas a blood vessel density was enhanced to 59.6±4.5/mm² by administration of a human recombinant HGF at 10 µg/ulcer/day (Fig. 1B). Therefore, it was revealed that a human recombinant HGF promotes neovascularization.

### (Result-2) Promotion of granulation tissue formation by human recombinant HGF

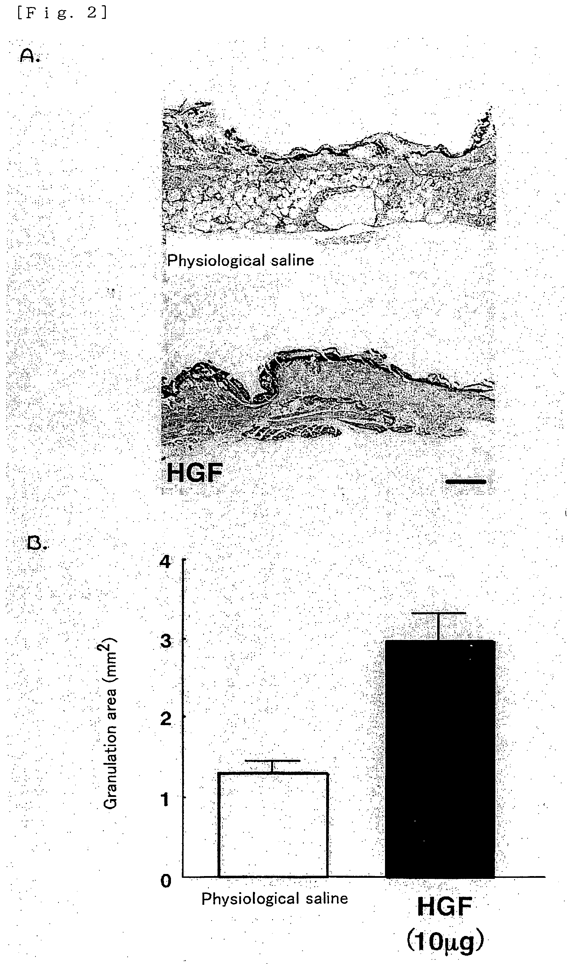

The effect of a human recombinant HGF on promotion of granulation tissue formation was investigated (Fig. 2A). Granulation tissue formation was measured by image analysis using a tissue section 7 days after producing a skin ulcer model. As a result, when a tissue after producing a skin ulcer model was investigated, Promotion of granulation tissue formation was recognized by administration of a human recombinant HGF (Fig. 2A). As a result of image analysis, an area of a granulation tissue was 1.30±0.15 mm² in a control group (physiological saline-administered group), whereas it was recognized that a human recombinant HGF enhances an area of a granulation tissue in a dose-dependent manner, an area of the granulation tissue was increased to 2.3-fold of a control by administration of a human recombinant HGF at 10 µg/ulcer/day, and a human recombinant HGF promotes the granulation tissue formation (Fig. 2B).

### (Result-3) Promotion of enclosure (healing) of ulcer part by human recombinant HGF

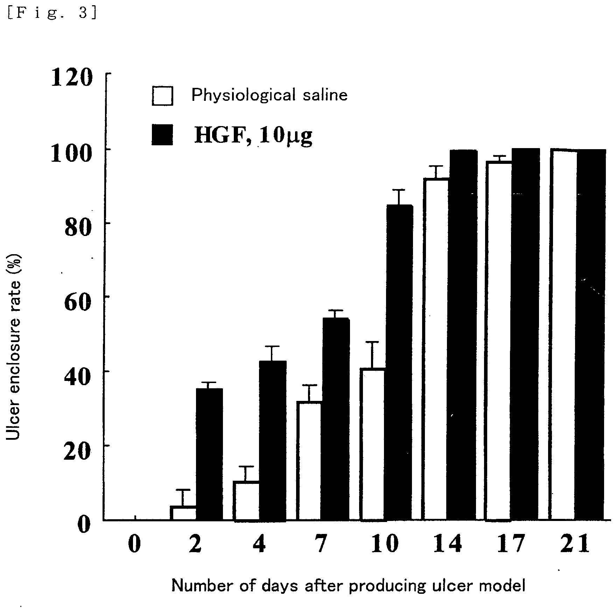

A human recombinant HGF (10 µg/ulcer/day) was administered to a skin ulcer part for consecutive five days. As a result, dose-dependent spur of enclosure (healing) of an ulcer part by the human tissue HGF was recognized from the two days after. Ten days after producing an ulcer model, in a control skin to which a physiological saline had been administered, an ulcer enclosure rate (healing rate) was 41.1±6.8%. To the contrary, an ulcer enclosure rate (healing rate) was enhanced to 85.0 ±4. 0% by administration of a human recombinant HGF at 10 µg/ulcer/day. In a control (physiological saline-administered group), completion of enclosure (healing) at an ulcer part took 21 days, whereas completion of enclosure (healing) of an ulcer part was recognized on the 14th day by administration of a human recombinant HGF at 10 µg/ulcer/day (Fig. 3).
<110> Kringle pharma CO., LTD.
   <110> NAKAMURA Toshikazu
<120> A pharmaceutical composition for treating or preventing cutaneous ulcer, which comprises recombinant human HGF.
<130> K13F1393
<160> 3
<210> 1
   <211> 723
   <212> PRT
   <213> Homo sapiens
<400> 1
<210> 2
   <211> 2172
   <212> DNA
   <213> Homo sapiens
<400> 2
   atgtgggtga ccaaactcct gccagccctg ctgctgcagc atgtcctcct gcatctcctc      60
   ctgctcccca tcgccatccc ctatgcagag ggacaaagga aaagaagaaa tacaattcat     120
   gaattcaaaa aatcagcaaa gactacccta atcaaaatag atccagcact gaagataaaa     180
   accaaaaaag tgaatactgc agaccaatgt gctaatagat gtactaggaa taaaggactt     240
   ccattcactt gcaaggcttt tgtttttgat aaagcaagaa aacaatgcct ctggttcccc     300
   ttcaatagca tgtcaagtgg agtgaaaaaa gaatttggcc atgaatttga cctctatgaa     360
   aacaaagact acattagaaa ctgcatcatt ggtaaaggac gcagctacaa gggaacagta     420
   tctatcacta agagtggcat caaatgtcag ccctggagtt ccatgatacc acacgaacac     480
   agctatcggg gtaaagacct acaggaaaac tactgtcgaa atcctcgagg ggaagaaggg     540
   ggaccctggt gtttcacaag caatccagag gtacgctacg aagtctgtga cattcctcag     600
   tgttcagaag ttgaatgcat gacctgcaat ggggagagtt atcgaggtct catggatcat     660
   acagaatcag gcaagatttg tcagcgctgg gatcatcaga caccacaccg gcacaaattc     720
   ttgcctgaaa gatatcccga caagggcttt gatgataatt attgccgcaa tcccgatggc     780
   cagccgaggc catggtgcta tactcttgac cctcacaccc gctgggagta ctgtgcaatt     840
   aaaacatgcg ctgacaatac tatgaatgac actgatgttc ctttggaaac aactgaatgc     900
   atccaaggtc aaggagaagg ctacaggggc actgtcaata ccatttggaa tggaattcca     960
   tgtcagcgtt gggattctca gtatcctcac gagcatgaca tgactcctga aaatttcaag    1020
   tgcaaggacc tacgagaaaa ttactgccga aatccagatg ggtctgaatc accctggtgt    1080
   tttaccactg atccaaacat ccgagttggc tactgctccc aaattccaaa ctgtgatatg    1140
   tcacatggac aagattgtta tcgtgggaat ggcaaaaatt atatgggcaa cttatcccaa    1200
   acaagatctg gactaacatg ttcaatgtgg gacaagaaca tggaagactt acatcgtcat    1260
   atcttctggg aaccagatgc aagtaagctg aatgagaatt actgccgaaa tccagatgat    1320
   gatgctcatg gaccctggtg ctacacggga aatccactca ttccttggga ttattgccct    1380
   atttctcgtt gtgaaggtga taccacacct acaatagtca atttagacca tcccgtaata    1440
   tcttgtgcca aaacgaaaca attgcgagtt gtaaatggga ttccaacacg aacaaacata    1500
   ggatggatgg ttagtttgag atacagaaat aaacatatct gcggaggatc attgataaag    1560
   gagagttggg ttcttactgc acgacagtgt ttcccttctc gagacttgaa agattatgaa    1620
   gcttggcttg gaattcatga tgtccacgga agaggagatg agaaatgcaa acaggttctc    1680
   aatgtttccc agctggtata tggccctgaa ggatcagatc tggttttaat gaagcttgcc    1740
   aggcctgctg tcctggatga ttttgttagt acgattgatt tacctaatta tggatgcaca    1800
   attcctgaaa agaccagttg cagtgtttat ggctggggct acactggatt gatcaactat    1860
   gatggcctat tacgagtggc acatctctat ataatgggaa atgagaaatg cagccagcat    1920
   catcgaggga aggtgactct gaatgagtct gaaatatgtg ctggggctga aaagattgga    1980
   tcaggaccat gtgaggggga ttatggtggc ccacttgttt gtgagcaaca taaaatgaga    2040
   atggttcttg gtgtcattgt tcctggtcgt ggatgtgcca ttccaaatcg tcctggtatt    2100
   tttgtccgag tagcatatta tgcaaaatgg atacacaaaa ttattttaac atataaggta    2160
   ccacagtcat ag                                                        2172
<210> 3
   <211> 728
   <212> PRT
   <213> Homo sapiens
<400> 3

## Claims

1. A composition for use in the promotion of the enclosure of skin ulcer by promoting the granulation formation and neovascularization, comprising a human recombinant HGF wherein five amino acid residues are deleted in the first Kringle domain, and/or a gene encoding said human recombinant HGF, wherein the human recombinant HGF in which five amino acid residues are deleted in the first Kringle domain is any one of the following:
(a) a protein comprising an amino acid sequence described in SEQ ID NO: 1 of the Sequence Listing;
(b) a protein comprising an amino acid sequence in which one to several amino acid(s) is/are deleted, substituted or added in SEQ ID NO: 1 of the Sequence Listing, and having the HGF activity; or
(c) a protein encoded by a DNA comprising a nucleotide sequence which has 90% or more homology with a nucleotide sequence represented by SEQ ID NO: 2 of the Sequence Listing, and having the HGF activity.

2. The composition for use according to claim 1, wherein the gene encoding a human recombinant HGF in which five amino acid residues are deleted in the first Kringle domain is a gene comprising any one of the following DNAs:
(a) a DNA comprising a nucleotide sequence described in SEQ ID NO: 2 of the Sequence listing; or
(b) a DNA comprising a nucleotide sequence in which one to several nucleotide(s) is/are deleted, substituted or added in SEQ ID NO: 2 of the Sequence Listing, and encoding a protein having the HGF activity.

3. The composition for use according to any of claims 1 or 2, wherein the skin ulcer is skin ulcer resulting from diabetes.

4. Use of a human recombinant HGF wherein five amino acid residues are deleted in the first Kringle domain and/or a gene encoding said human recombinant HGF, for preparing a drug for promoting the enclosure of skin ulcer by promoting the granulation formation and neovascularization, wherein the human recombinant HGF in which five amino acid residues are deleted in the first Kringle domain is any one of the following:
(a) a protein comprising an amino acid sequence described in SEQ ID NO: 1 of the Sequence Listing;
(b) a protein comprising an amino acid sequence in which one to several amino acid(s) is/are deleted, substituted or added in SEQ ID NO: 1 of the Sequence Listing, and having the HGF activity; or
(c) a protein encoded by a DNA comprising a nucleotide sequence which has 90% or more homology with a nucleotide sequence represented by SEQ ID NO: 2 of the Sequence Listing, and having the HGF activity.

5. The use according to claim 4, wherein the gene encoding a human recombinant HGF in which five amino acid residues are deleted in the first Kringle domain is a gene comprising any one of the following DNAs:
(a) a DNA comprising a nucleotide sequence described in SEQ ID NO: 2 of the Sequence Listing;
(b) a DNA comprising a nucleotide sequence in which one to several nucleotide(s) is/are deleted, substituted or added in SEQ ID NO: 2 of the Sequence Listing, and encoding a protein having the HGF activity.

6. The use according to claim 4 or 5, wherein the skin ulcer is skin ulcer resulting from diabetes.

7. A sealing-type skin ulcer covering material, comprising a human recombinant HGF wherein five amino acid residues are deleted in the first Kringle domain, wherein the human recombinant HGF in which five amino acid residues are deleted in the first Kringle domain is any one of the following:
(a) a protein comprising an amino acid sequence described in SEQ ID NO: 1 of the Sequence Listing;
(b) a protein comprising an amino acid sequence in which one to several amino acid(s) is/are deleted, substituted or added in SEQ ID NO: 1 of the Sequence Listing, and having the HGF activity; or
(c) a protein encoded by a DNA comprising a nucleotide sequence which has 90% or more homology with a nucleotide sequence represented by SEQ ID NO: 2 of the Sequence Listing, and having the HGF activity.

8. A kit for promoting the enclosure of a skin ulcer by promoting the granulation formation and neovascularization, comprising a composition containing a human recombinant HGF wherein five amino acid residues are deleted in the first Kringle domain, and a sealing-type wound covering material which can absorb an exudate from an affected part of skin ulcer, wherein the human recombinant HGF in which five amino acid residues are deleted in the first Kringle domain is any one of the following:
(a) a protein comprising an amino acid sequence described in SEQ ID NO: 1 of the Sequence Listing;
(b) a protein comprising an amino acid sequence in which one to several amino acid(s) is/are deleted, substituted or added in SEQ ID NO: 1 of the Sequence Listing, and having the HGF activity; or
(c) a protein encoded by a DNA comprising a nucleotide sequence which has 90% or more homology with a nucleotide sequence represented by SEQ ID NO: 2 of the Sequence Listing, and having the HGF activity.

## Patentansprüche

1. Zusammensetzung für die Verwendung zur Förderung des Verschließens von Haut-Ulcus durch Fördern des Granulationsaufbaus und der Neovaskularisierung, die einen humanen rekombinanten HGF aufweist, wobei fünf Aminosäurereste in der ersten Kringle-Domain deletiert sind, und/oder ein Gen aufweist, das für den humanen rekombinanten HGF codiert, wobei der humane rekombinante HGF, in dem fünf Aminosäurereste in der ersten Kringle-Domain deletiert sind, aus Folgendem ausgewählt ist:
(a) einem Protein, das eine Aminosäuresequenz aufweist, die in der SEQ ID NO. 1 des Sequenzprotokolls beschrieben wird;
(b) einem Protein, das eine Aminosäuresequenz aufweist, in der eine bis mehrere Aminosäure(n) in der SEQ ID NO. 1 des Sequenzprotokolls deletiert, substituiert oder hinzugefügt ist/sind, und das die HGF-Aktivität aufweist; oder
(c) einem Protein, das von einer DNA codiert wird, die eine Nukleotidsequenz aufweist, die 90% oder mehr Homologie mit einer Nukleotidsequenz teilt, die durch die SEQ ID NO. 2 des Sequenzprotokolls repräsentiert wird, und die HGF-Aktivität aufweist.

2. Zusammensetzung zur Verwendung gemäß Anspruch 1, wobei das Gen, das für einen humanen rekombinanten HGF codiert, in dem fünf Aminosäurereste in der ersten Kringle-Domain deletiert sind, ein Gen ist, das eine der folgenden DNAs aufweist:
(a) eine DNA, die eine Nukleotidsequenz aufweist, die in der SEQ ID NO. 2 des Sequenzprotokolls beschrieben ist; oder
(b) eine DNA, die eine Nukleotidsequenz aufweist, in der eine bis mehrere Nukleotid(e) in der SEQ ID NO. 2 des Sequenzprotokolls deletiert, substituiert oder hinzugefügt ist/sind, und für ein Protein codiert, das die HGF-Aktivität aufweist.

3. Zusammensetzung zur Verwendung gemäß einem der Ansprüche 1 oder 2, wobei das Haut-Ulcus ein solches Haut-Ulcus ist, das sich aus einem Diabetes ergibt.

4. Verwendung eines humanen rekombinanten HGF, wobei fünf Aminosäurereste in der ersten Kringle-Domain deletiert sind, und/oder eines Gens, das für den humanen rekombinanten HGF codiert, zur Herstellung eines Arzneimittels, um das Verschließen von Haut-Ulcus zu fördern, indem der Granulationsaufbau und die Neovaskularisierung gefördert werden, wobei der humane rekombinante HGF, in dem fünf Aminosäurereste in der ersten Kringle-Domain deletiert sind, aus Folgendem ausgewählt ist:
(a) einem Protein, das eine Aminosäuresequenz aufweist, die in der SEQ ID NO. 1 des Sequenzprotokolls beschrieben ist;
(b) einem Protein, das eine Aminosäuresequenz aufweist, in der eine bis mehrere Aminosäure(n) in der SEQ ID NO. 1 des Sequenzprotokolls deletiert, substituiert oder hinzugefügt ist/sind, und das die HGF-Aktivität aufweist; oder
(c) einem Protein, das von einer DNA codiert wird, die eine Nukleotidsequenz aufweist, die 90% oder mehr Homologie mit einer Nukleotidsequenz teilt, die durch die SEQ ID NO. 2 des Sequenzprotokolls repräsentiert wird, und das die HGF-Aktivität aufweist.

5. Verwendung gemäß Anspruch 4, wobei das Gen, das einen humanen rekombinanten HGF codiert, in dem fünf Aminosäurereste in der ersten Kringle-Domain deletiert sind, ein Gen ist, das eine der folgenden DNAs aufweist:
(a) eine DNA, die eine Nukleotidsequenz aufweist, die in der SEQ ID NO. 2 des Sequenzprotokolls beschrieben wird;
(b) eine DNA, die eine Nukleotidsequenz aufweist, in der ein bis mehrere Nukleotid(e) in der SEQ ID NO. 2 des Sequenzprotokolls deletiert, subsituiert oder hinzugefügt ist/sind, und die für ein Protein mit HGF-Aktivität codiert.

6. Verwendung gemäß Anspruch 4 oder 5, wobei das Haut-Ulcus ein solches Haut-Ulcus ist, das aus einem Diabetes resultiert.

7. Abdeckmaterial vom Dichtungstyp für Haut-Ulcus, das einen humanen rekombinanten HGF aufweist, wobei fünf Aminosäurereste in der ersten Kringle-Domain deletiert sind, wobei der humane rekombinante HGF, in dem fünf Aminosäurereste in der ersten Kringle-Domain deletiert sind, aus Folgendem ausgewählt ist:
(a) einem Protein, das eine Aminosäuresequenz aufweist, die in der SEQ ID NO. 1 des Sequenzprotokolls beschrieben wird;
(b) einem Protein, das eine Aminosäuresequenz aufweist, in der eine bis mehrere Aminosäure(n) in der SEQ ID NO. 1 des Sequenzprotokolls deletiert, substituiert oder hinzugefügt ist/sind, und das die HGF-Aktivität aufweist; oder
(c) einem Protein, das von einer DNA codiert wird, die eine Nukleotidsequenz aufweist, die 90% oder mehr Homologie mit einer Nukleotidsequenz teilt, die durch die SEQ ID NO. 2 des Sequenzprotokolls repräsentiert wird, und das die HGF-Aktivität aufweist.

8. Kit zum Fördern des Umschließens von Haut-Ulcus durch Fördern des Granulationsaufbaus und der Neovaskularisierung, das eine Zusammensetzung aufweist, die einen humanen rekombinanten HGF enthält, wobei fünf Aminosäurereste in der ersten Kringle-Domain deletiert sind, und ein Abdeckmaterial für Wunden vom Dichtungstyp, das ein Exsudat von einem Bereich, das von Haut-Ulcus betroffen ist, absorbieren kann aufweist, wobei der humane rekombinante HGF, in dem fünf Aminosäurereste in der ersten Kringle-Domain deletiert sind, aus Folgendem ausgewählt ist:
(a) einem Protein, das eine Aminosäuresequenz aufweist, die in der SEQ ID NO. 1 des Sequenzprotokolls beschrieben wird;
(b) einem Protein, das eine Aminosäuresequenz aufweist, in der eine bis mehrere Aminosäure(n) in der SEQ ID NO. 1 des Sequenzprotokolls deletiert, substituiert oder hinzugefügt ist/sind, und das die HGF-Aktivität aufweist; oder
(c) einem Protein, das von einer DNA codiert ist, die eine Nukleotidsequenz aufweist, die 90% oder mehr Homologie mit einer Nukleotidsequenz teilt, die durch die SEQ ID NO. 2 des Sequenzprotokolls repräsentiert wird, und das die HGF-Aktivität aufweist.

## Revendications

1. Composition à utiliser pour favoriser la fermeture de l'ulcère cutané en favorisant la formation de granulation et la néovascularisation, comprenant un HGF recombinant humain où cinq résidus d'acides aminés sont supprimés dans le premier domaine Kringle, et/ou un gène codant pour ledit HGF recombinant humain, où le HGF recombinant humain dans lequel cinq résidus d'acides aminés sont supprimés dans le premier domaine Kringle est l'une quelconque des protéines suivantes :
(a) une protéine comprenant une séquence d'acides aminés décrite dans la séquence SEQ ID NO : 1 de la liste des séquences ;
(b) une protéine comprenant une séquence d'acides aminés dans laquelle un ou plusieurs acide(s) aminé(s) est/sont supprimé(s), substitué(s) ou ajouté(s) dans la séquence SEQ ID NO : 1 de la liste des séquences, et ayant l'activité HGF ; et
(c) une protéine codée par un ADN comprenant une séquence nucléotidique qui présente une homologie de 90% ou plus avec une séquence nucléotidique représentée par la séquence SEQ ID NO : 2 de la liste des séquences, et ayant l'activité HGF.

2. Composition pour une utilisation selon la revendication 1, dans laquelle le gène codant pour un HGF recombinant humain dans lequel cinq résidus d'acides aminés sont supprimés dans le premier domaine Kringle est un gène comprenant l'un quelconque des ADN suivants :
(a) un ADN comprenant une séquence nucléotidique décrite dans la séquence SEQ ID NO : 2 de la liste des séquences ; et
(b) un ADN comprenant une séquence nucléotidique dans laquelle un ou plusieurs nucléotide(s) est/sont supprimé(s), substitué(s) ou ajouté(s) dans la séquence SEQ ID NO : 2 de la liste des séquences, et codant pour une protéine ayant l'activité HGF.

3. Composition pour une utilisation selon l'une des revendications 1 et 2, dans laquelle l'ulcère cutané est un ulcère cutané résultant du diabète.

4. Utilisation d'un HGF recombinant humain où cinq résidus d'acides aminés sont supprimés dans le premier domaine Kringle et/ou d'un gène codant pour ledit HGF recombinant humain, pour préparer un médicament pour favoriser la fermeture de l'ulcère cutané en favorisant la formation de granulation et la néovascularisation, où le HGF recombinant humain dans lequel cinq résidus d'acides aminés sont supprimés dans le premier domaine Kringle est l'une quelconque des protéines suivantes :
(a) une protéine comprenant une séquence d'acides aminés décrite dans la séquence SEQ ID NO : 1 de la liste des séquences ;
(b) une protéine comprenant une séquence d'acides aminés dans laquelle un ou plusieurs acide(s) aminé(s) est/sont supprimé(s), substitué(s) ou ajouté(s) dans la séquence SEQ ID NO : 1 de la liste des séquences, et ayant l'activité HGF ; et
(c) une protéine codée par un ADN comprenant une séquence nucléotidique qui présente une homologie de 90% ou plus avec une séquence nucléotidique représentée par la séquence SEQ ID NO : 2 de la liste des séquences, et ayant l'activité HGF.

5. Utilisation selon la revendication 4, dans laquelle le gène codant pour un HGF recombinant humain dans lequel cinq résidus d'acides aminés sont supprimés dans le premier domaine Kringle est un gène comprenant l'un quelconque des ADN suivants :
(a) un ADN comprenant une séquence nucléotidique décrite dans la séquence SEQ ID NO : 2 de la liste des séquences ;
(b) un ADN comprenant une séquence nucléotidique dans laquelle un ou plusieurs nucléotide(s) est/sont supprimé(s), substitué(s) ou ajouté(s) dans la séquence SEQ ID NO : 2 de la liste des séquences, et codant pour une protéine ayant l'activité HGF.

6. Utilisation selon la revendication 4 ou 5, dans laquelle l'ulcère cutané est un ulcère cutané résultant du diabète.

7. Matériau de recouvrement d'ulcère cutané de type étanche, comprenant un HGF recombinant humain dans lequel cinq résidus d'acides aminés sont supprimés dans le premier domaine Kringle, où le HGF recombinant humain dans lequel cinq résidus d'acides aminés sont supprimés dans le premier domaine Kringle est l'une quelconque des protéines suivantes :
(a) une protéine comprenant une séquence d'acides aminés décrite dans la séquence SEQ ID NO : 1 de la liste des séquences ;
(b) une protéine comprenant une séquence d'acides aminés dans laquelle un ou plusieurs acide(s) aminé(s) est/sont supprimé(s), substitué(s) ou ajouté(s) dans la séquence SEQ ID NO : 1 de la liste des séquences, et ayant l'activité HGF ; et
(c) une protéine codée par un ADN comprenant une séquence nucléotidique qui présente une homologie de 90% ou plus avec une séquence nucléotidique représentée par la séquence SEQ ID NO : 2 de la liste des séquences, et ayant l'activité HGF.

8. Kit pour favoriser la fermeture d'un ulcère cutané en favorisant la formation de granulation et la néovascularisation, comprenant une composition contenant un HGF recombinant humain dans lequel cinq résidus d'acides aminés sont supprimés dans le premier domaine Kringle, et un matériau de recouvrement de plaie de type étanche qui peut absorber un exsudat d'une partie affectée d'un ulcère cutané, où le HGF recombinant humain dans lequel cinq résidus d'acides aminés sont supprimés dans le premier domaine Kringle est l'une quelconque des protéines suivantes :
(a) une protéine comprenant une séquence d'acides aminés décrite dans la séquence SEQ ID NO : 1 de la liste des séquences ;
(b) une protéine comprenant une séquence d'acides aminés dans laquelle un ou plusieurs acide(s) aminé(s) est/sont supprimé(s), substitué(s) ou ajouté(s) dans la séquence SEQ ID NO : 1 de la liste des séquences, et ayant l'activité HGF ; et
(c) une protéine codée par un ADN comprenant une séquence nucléotidique qui présente une homologie de 90% ou plus avec une séquence nucléotidique représentée par la séquence SEQ ID NO : 2 de la liste des séquences, et ayant l'activité HGF.
